# EUROPÄISCHE PATENTSCHRIFT

(11) **EP 2 535 019 B1**
(45) Veröffentlichungstag und Bekanntmachung des Hinweises auf die Patenterteilung: **14.02.2018**
(21) Anmeldenummer: 11169929.4
(22) Anmeldetag: 15.06.2011
(51) Int. Cl.: A61F 2/16

(54) **AKKOMMODATIVE INTRAOKULARE PHAKE KUNSTLINSE MIT HOCHBRECHENDEM MEDIUM**
ACCOMMODATIVE INTRAOCULAR PHAKIC ARTIFICIAL LENS WITH HIGHLY REFRACTIVE MEDIUM
LENTILLE ARTIFICIELLE INTRAOCULAIRE PHAKE ACCOMMODATIVE DOTÉE D'UN SUPPORT TRÈS RÉFRINGENT

(43) Veröffentlichungstag der Anmeldung: 19.12.2012
(73) Patentinhaber: Detmers, Ulfert, 26506 Norden (DE); Meyer, Matthias, 26810 Westoverledingen (DE)
(72) Erfinder: Detmers, Ulfert, 26506 Norden (DE); Meyer, Matthias, 26810 Westoverledingen (DE)
(74) Vertreter: Kaminski Harmann

(56) Entgegenhaltungen:
- EP-A1- 1 958 592
- WO-A1-2008/101522
- US-A- 4 731 078
- US-A1- 2008 046 076

## Beschreibung

Die Erfindung betrifft eine akkommodative Intraokularlinse für phake Augen, d.h. für Augen, die eine natürliche oder naturentsprechende Linse enthalten, wie dies im Oberbegriff des Anspruchs 1 ausgedrückt ist.

Im Weiteren sollen unter dem Begriff natürliche Linse, im Auge befindliche natürliche biologische Linsen und naturentsprechende künstliche Linsen verstanden werden.

Zur Korrektur von Refraktionsfehlern des menschlichen Auges sind im Stand der Technik zwei Arten von Kunstlinsen bekannt. Die eine Art Kunstlinsen wird als Ersatz für die natürliche Linse ins Auge eingesetzt, nachdem die natürliche Linse durch Extraktion komplett aus dem Auge entfernt worden ist. Naturgemäß sind solche Linsen entsprechend groß und die Extraktion der natürlichen Linse sowie das Einsetzen der Ersatzlinse stellen einen tiefgreifenden Eingriff dar. Daher wird ein solcher Eingriff in der Regel nur bei einem ausgeprägten grauen Star durchgeführt, der normalerweise erst jenseits des 60sten Lebensjahres auftritt. Die implantierten Ersatzlinsen, müssen nicht nur die Aufgaben der natürlichen Linse in Bezug auf ihre Brechungseigenschaften (Refraktion) sondern auch in Bezug auf ihre akkommodativen Fähigkeiten (Scharfstellen bei Fern- und Nahsicht) übernehmen.

Eine solche Ersatzlinse ist beispielsweise aus EP 0 356 050 A1 bekannt. Die beschriebene intraokulare Ersatzlinse wird anstelle der natürlichen Linse ins Auge implantiert und weist einen Linsenkörper auf, der im implantierten Zustand im Strahlengang liegt und in den ein mit einer Flüssigkeit füllbarer Raum integriert ist. Gegenüber dem posterioren Augeninneren wird der füllbare Raum durch einen steifen Linsenteil abgegrenzt, gegenüber dem anterioren Kammerwasser des Auges durch eine elastische Membran, die ihren Krümmungsradius abhängig vom Füllgrad des Raumes ändert, wodurch sich die Konvexität der Ersatzlinse und also ihre optische Geometrie ändert. Außerdem weist die Linse an der Peripherie des Linsenkörpers eine im implantierten Zustand außerhalb des Strahlenganges liegende Haptik auf. In die Haptik sind flexible, blasenähnliche Behältnisse integriert, die mit einer Flüssigkeit gefüllt sind und mit dem füllbaren Raum des Linsenkörpers in Strömungskontakt stehen. Der Brechungsindex der Flüssigkeit liegt über dem der umgebenden Medien. Die Haptik wird bei der Implantation mit der inneren Augenmuskulatur verbunden. Bei Kontraktion der inneren Augenmuskulatur wird die Flüssigkeit aus den blasenähnlichen Behältnissen der Haptik in den dafür vorgesehenen Raum im Linsenkörper gedrückt und so dessen Füllgrad und die Konvexität der elastischen Membran verändert, wodurch sich auch die Brechkraft ändert.

Diese Kunstlinse ist sehr groß und weist einen sehr kompliziert aufgebauten Apparat mit blasenähnlichen Behältnissen und Strömungsleitungen auf, um eine Akkommodation zu realisieren. Der für die Akkommodation nötige Apparat ist in der Haptik integriert, die darum ebenfalls einen entsprechenden Platzbedarf aufweist. Um diese Kunstlinse verwenden zu können, muss daher die natürliche Linse extrahiert werden, da beide Linsen zusammen im Auge keinen Platz fänden. Außerdem wäre eine ausreichende Verbindung der Haptik mit der inneren Augenmuskulatur bei Vorhandensein der natürlichen Linse im Auge nicht möglich, so dass die Funktion des durch die Augenmuskulatur gesteuerten komplizierten Apparats zur Realisierung der Akkommodation nicht mehr gewährleistet werden könnte.

Die andere Art intraokularer Kunstlinsen, die sogenannten Phaken Linsen, werden zusätzlich zur natürlichen Augenlinse ins Auge implantiert. Diese intraokularen Kunstlinsen sollen Refraktionsfehler, wie zum Beispiel Kurzsichtigkeit oder Weitsichtigkeit ausgleichen und eine Brille ersetzen, bzw. verträglicher machen, wobei die Einpflanzung üblicherweise über einen Hornhautschnitt mit faltbaren oder starren Kunstlinsen erfolgt. Phake Linsen werden üblicherweise entweder vor der Iris als sogenannte Vorderkammerlinsen oder zwischen Iris und natürlicher Linse als sogenannte Hinterkammerlinsen implantiert. Der Eingriff ist weit weniger gravierend und wird daher bereits bei jüngeren Erwachsenen durchgeführt.

Bekannte Phake Linsen, die zur Implantation zusätzlich zur natürlichen Linse ins Auge verwendet werden, sind aus Silikon, Hydrogel oder aus starren wie flexiblen Acrylaten gebildet. Da sie zusätzlich zur noch vorhandenen natürlichen Linse ins Auge implantiert werden, sind sie in der Regel kleiner als die oben beschriebenen Ersatzlinsen. Sie weisen ohne Haptik einen Durchmesser von 6mm bis 7mm auf. Bislang wurde mit Phaken Linsen in der Regel nur ein Refraktionsfehler ausgeglichen. Die im Alter zunehmende Akkommodationsschwäche (Alterssichtigkeit / Presbyopie) wurde jedoch durch Phake Linsen nicht korrigiert, so daß trotz Korrektur des Refraktionsfehlers im Alter wieder eine zusätzliche Korrektur, z. B. durch eine Brille erforderlich wurde.

Aus WO 2008/101522 A1 ist jedoch eine Phake Linse bekannt, die auch eine altersbedingte Akkommodationsschwäche ausgleichen kann. Diese Phake Intraokularlinse weist einen Linsenkörper aus optisch transparentem Linsenmaterial mit einem ersten flächigen Linsenteil und einem gegenüberliegenden zweiten flächigen Linsenteil auf. Der erste Linsenteil ist zur Kontaktierung der natürlichen Linse des menschlichen Auges ausgebildet und weist gegenüber dem zweiten Linsenteil eine höhere Deformierbarkeit auf. Der starre zweite Linsenteil ist der Regenbogenhaut zugewandt und kann eine Refraktionskorrektur aufweisen. Zwischen dem ersten Linsenteil und dem zweiten Linsenteil ist ein Hohlraum im Linsenkörper ausgebildet, der mit einem Fluidum - vorzugsweise Gas - gefüllt ist. Der Brechungsindex des Fluidums ist erheblich geringer als der der umgebenden Medien. Zur Verstärkung der akkommodativen Funktion der im Auge belassenen natürlichen Linse wird die Änderung des Krümmungsradius der natürlichen Linse auf den an ihr anliegenden ersten Linsenteil der künstlichen Linse übertragen. Die Konkavität des ersten Linsenteils wird dadurch erhöht, während der der Iris zugewandte zweite Linsenteil in seiner Form unverändert bleibt. Die Zunahme der Konkavität zusammen mit dem geringeren Brechungsindex des im Hohlraum befindlichen Mediums bewirken schließlich eine Verstärkung des akkommodativen Effekts der natürlichen Linse. Obwohl sich die in WO 2008/101522 A1 offenbarte Kunstlinse ein einfaches physikalisches Prinzip zu Nutze macht, ist die Herstellung einer solchen Linse wegen der speziellen Anforderungen an die Materialien relativ teuer und schwierig.

Die vorliegende Erfindung gemäß Anspruch 1 betrifft eine Phake Linse, die dazu ausgebildet ist, in Kontakt mit der natürlichen Linse ins Auge implantiert zu werden, und die insbesondere zur Presbyopiekorrektur dient und stellt eine verbesserte Alternative zu der bekannten Phaken Linse dar. Sie lässt sich insbesondere auch einfacher und kostengünstiger herstellen.

Mit der Phaken Intraokularlinse gemäß Anspruch 1 bzw. durch eine Weiterbildung dieser Phaken Intraokularlinse gemäß einem der abhängigen Ansprüche wird eine intraokulare Phake Kunstlinse zur Verfügung gestellt, die, zusätzlich zur natürlichen Linse in das Auge implantiert, sowohl den Mangel der Akkommodation durch Alter in erheblichem Maße reduziert als auch - bei Bedarf - optische Refraktionsfehler ausgleicht. Dadurch wird dem Auge sehr lange eine hohe Sehschärfe erhalten, ohne daß weitere Hilfsmittel wie Brillen oder ähnliches nötig werden.

Erfindungsgemäß wird dabei die Akkommodationsfähigkeit des Auges und seiner natürlichen Linse, die auch im Alter noch vorhanden sind, genutzt. Die optischen und mechanischen Eigenschaften der erfindungsgemäßen Kunstlinse erhöhen dabei die Effektivität der im Alter reduzierten Akkommodation um ein Vielfaches. Dies wird dadurch erreicht, daß die Form eines hochbrechenden flexiblen Lenticels, der in einer zumindest teilweise flexiblen Kunstlinse eingeschlossen ist, durch die Bewegung der Restakkommodation verändert wird.

Grundsätzlich bestimmt die Differenz der Brechungsindizes der einzelnen optischen Elemente die Größe der Gesamtbrechkraft eines optischen Systems. Für die natürliche Linse im Kontakt mit dem umgebenden Kammerwasser liegt die Differenz der Brechungsindizes bei ca. 0,07. Wird die natürliche Linse mit einer formveränderbaren künstlichen Phaken Linse ergänzt, die ein Lenticel mit einem viskosen Medium mit abweichendem Brechungsindex umfasst, so kann die Differenz der Brechungsindizes zwischen Kammerwasser, natürlicher Linse und künstlicher Linse materialabhängig bei deutlich höheren Werten liegen. Entsprechend wird eine akkommodative Linsenwölbung, die bei kleiner Brechungsindexdifferenz von 0,07 nur eine Brechkraftänderung von 0,5 Dioptrien bewirkt, bei einer Indexdifferenz von beispielsweise 0,15 deutlich effizienter.

Bei der erfindungsgemäßen Phaken Intraokularlinse wird die nötige Brechkraftänderung für eine Nahakkommodation durch eine Materialverlagerung des im Linseninneren befindlichen hochbrechenden Mediums des Lenticels bewirkt. Dabei ist es unerheblich, ob der Lenticel zunächst konstruktionsbedingt eine konkave, konvexe oder flächenparallele Form besitzt. In der Regel erfolgt die Materialverlagerung des Lenticelmaterials von der Peripherie ins Zentrum der Linse, wodurch eine Erhöhung der Konvexität erreicht wird. Für zunächst konkave Lenticel muss diese Aussage allerdings bedingt eingeschränkt werden. Für konkave Lenticel gilt, daß sie zur Brechkrafterhöhung der intraokularen Kunstlinse in der Regel peripher zunehmend abgeflacht werden, um die gewünschte Erhöhung der Konvexität zu erreichen. Hierbei muss eine zentrale Materialverlagerung nicht zwingend stattfinden, sondern es ist eine Materialverlagerung in weiter peripher gelegene Linsenkompartimente möglich. Die konkave Linse wird weniger konkav.

Die erfindungsgemäße Phake Intraokularlinse ist für die angestrebte Verstärkung der Akkommodationsfähigkeit des Auges aus optisch transparentem Material gebildet und umfasst einen ersten flächigen Linsenteil, der im implantierten Zustand posterior angeordnet ist, einen dem ersten Linsenteil im implantierten Zustand anterior gegenüberliegenden zweiten flächigen Linsenteil sowie einen zwischen diesen Linsenteilen gelegenen Hohlraum, der ein optisch transparentes Medium umschließt. Das Medium ist Bestandteil eines Lenticels, weist einen Brechungsindex auf der höher ist als derjenige der umgebenden Medien, und stellt ein im Hohlraum verlagerbares Lenticelmaterial dar.

Der erste Linsenteil ist so ausgebildet, daß er im implantierten Zustand an der natürlichen Linse des menschlichen Auges anliegen kann. Er weist eine Deformierbarkeit auf, die eine möglichst enge und direkte Folgebewegung zur akkommodativen Aktion der natürlichen Linse ermöglicht. Der zweite Linsenteil umfasst zumindest einen deformierbaren und/oder dehnbaren Teilbereich, der initiiert durch die akkommodative Bewegung der natürlichen Linse seine Form bzw. seinen Dehnungszustand ändern kann.

Die für den ersten Linsenteil und den zweiten Linsenteil gewählten Materialien haben vorzugsweise einen Brechungsindex der ungefähr dem des Kammerwassers entspricht.

Die erfindungsgemäße intraokulare Phake Linse hat somit wenigstens drei funktionell wirkende Teile: den verformbaren Lenticel aus einem hochbrechenden Medium; den an der natürlichen Linse anliegenden deformierbaren ersten Linsenteil; und den der natürlichen Linse abgewandten zweiten Linsenteil mit zumindest einem deformierbaren Bereich. Bei akkommodativen Aktionen des Auges passt sich der erste Linsenteil unmittelbar der Formveränderung der natürlichen Linse an. Durch diese Formveränderung des ersten Linsenteils wird eine Umverteilung des Lenticelmaterials im Hohlraum zwischen den Linsenteilen und zugleich eine Formveränderung des deformierbaren Bereichs des zweiten Linsenteils bewirkt.

Im Weiteren werden Eigenschaften wie Flexibilität und Dehnbarkeit als besondere Formen der Deformierbarkeit betrachtet. Der Begriff Deformierbarkeit wird somit im Weiteren als Oberbegriff verwendet und umfasst auch Eigenschaften wie Dehnbarkeit und Flexibilität.

Der Lenticel bildet eine optisch wirksame Linse im Gesamtsystem der Optik der Kunstlinse, wobei das Medium des Lenticels gasförmig, ölig, gelartig, flüssig oder deformierbar fest sein kann. Die Formveränderung des Lenticels wird durch die akkommodativ bedingte Zunahme der Krümmung der natürlichen Augenlinse initiiert.

Der Lenticel kann im wesentlichen aus dem Medium/Lenticelmaterial bestehen und nach außen durch den ersten Linsenteil und den zweiten Linsenteil begrenzt sein, so daß er durch den Hohlraum bzw. die Ausgestaltung der dem Hohlrum zugewandten Innenseiten des ersten Linsenteils und des zweiten Linsenteils bestimmt ist. Der Lenticel kann aber auch eine Membran aufweisen, die das Medium/Lenticelmaterial umschließt und den Lenticel nach außen abgrenzt. Die Membran kann so ausgestaltet sein, daß sie maßgeblich die äußere Form des Lenticels im Hohlraum zwischen den Linsenteilen bestimmt. Zusätzlich zu dem Lenticel mit seinem Medium und seiner Membran kann in einem solchen Fall ein weiteres Medium im Hohlraum zwischen den Linsenteilen vorgesehen sein. Das Medium des Lenticels sollte dann einen Brechungsindex aufweisen, der möglichst von dem der im Auge vorhandenen Medien abweicht, während das zusätzliche Medium einen möglichst ähnlichen Brechungsindex haben sollte wie die umgebenden Medien.

Durch Verwendung einer möglichst hochbrechenden Substanz als Medium des Lenticels, deren Brechungsindex mindestens einen Wert von 1,4 übertreffen sollte, kann der Lenticel durch Formveränderung die Brechkraft des gesamten Linsensystems deutlich stärker ändern, als es unter normalen Bedingungen der akkommodativen Formveränderung im Auge möglich ist.

Zur Erläuterung der sich bei Nahakkommodation in einem Auge mit implantierter erfindungsgemäßer Phaken Linse ablaufenden Prozesse wird zunächst davon ausgegangen, daß sich das Auge zusammen mit der oben beschriebenen erfindungsgemäßen Kunstlinse im nicht akkommodierten, bzw. entspannten Zustand befindet. Die Fokussierung sei für die Ferne eingestellt. Etwaige Brechungsfehler des Auges wie zum Beispiel Kurz- oder Weitsichtigkeit können durch die Form und/oder den Brechungsindex des Lenticels und/oder des ersten Linsenteils und/oder des zweiten Linsenteils ausgeglichen sein. Der erste Linsenteil liegt, vorzugsweise getrennt durch einen dünnen Flüssigkeitsfilm an der natürlichen Linse an. Bei einer Nahakkommodation kommt es nun zu einer Formveränderung der natürlichen Linse und der Radius der Vorderfläche der natürlichen Linse verringert sich um ca. 1mm bis 2mm. Hieraus ergibt sich eine Brechkraftänderung der natürlichen Linse von ca. 0,5 dpt bis 1,2 dpt. Die Formveränderung der natürlichen Linse überträgt sich auf die künstliche Linse.

Da die künstliche Intraokularlinse in ihrer Struktur in den verschiedenen Bereichen deutlich unterschiedlich flexible Zonen besitzt, kann sie sich, wenn sie der Bewegung der natürlichen Linse folgt, nicht gleichförmig kontinuierlich in ihrer Form verändern, sondern flexiblere Bereiche und das viskose Medium des Lenticels werden der Formveränderung erheblich stärker folgen als starre unflexible Bereiche.

Wird das viskose Medium im Hohlraum der Kunstlinse, mit einem deutlich höheren Brechungsindex als demjenigen des Kammerwassers gewählt und so gestaltet, daß es die Form einer Linse ergibt, die wenn sie der akkommodativen Bewegung der natürlichen Linse folgt, in Ihrer Brechkraft positiver wird, so ist eine Verstärkung der Restakkommodation die Folge. Diese Verstärkung ist umso größer, je höher die Differenz zwischen den Krümmungsradien der Vorderflächen der natürlichen Linse und des Lenticels ist, gemessen im nicht akkommodierten und akkommodierten Zustand.

Je nach Ausführungsform der erfindungsgemäßen Phaken Linse sind der erste Linsenteil und der zweite Linsenteil mit ihren verformbaren Bereichen konstruktiv so aufeinander abgestimmt, daß es bei einer Nahakkommodation der natürlichen Linse zu einer Materialverlagerung im Lenticel in einer solche Weise kommt, daß der Lenticel eine zunehmend konvexere Form annimmt. Aufgrund des hohen Brechungsindexes des Lenticelmaterials führt dies zu einer Brechkrafterhöhung der Phaken Intraokularlinse, die die akkommodativen Eigenschaften der natürlichen Linse verstärkt.

Insbesondere wird bei einer Nahakkommodation der an der natürlichen Linse anliegende erste Linsenteil konkaver, der der natürlichen Linse abgewandte zweite Linsenteil wird konvexer. Der Lenticel zwischen beiden Linsenteilen wird entsprechend in seiner Form verändert und trägt, wie oben beschrieben, über seinen Brechungsindex und die Verformung zur Verstärkung der Restakkommodation bei.

Je nach Ausführungsform der erfindungsgemäßen Phaken intraokularen Kunstlinse wird der zweite Linsenteil in einem solchen Maß konvexer, das die zunehmende Konkavität des ersten Linsenteils mehr als nur kompensiert wird. Das heißt die Konkavitätszunahme des ersten an der natürlichen Linse anliegenden Linsenteils wird bei der Nahakkommodation dazu genutzt, eine Konvexität im zweiten der natürlichen Linse abgewandten Linsenteil zu erzeugen, die - je nach Ausführungsform - die Konkavität ausgleicht oder erheblich übertrifft.

Da die hohen Anforderungen an die verwendeten Materialien, wie Langlebigkeit, hohe Diffusionsdichtigkeit über die gesamte Lebensdauer, Biokompatibilität für Materialien im Gebrauch zusammen mit einem Lenticelmaterial mit einem hohen Brechungsindex, schwerer zu erfüllen sind und solche Materialien teurer sind als für Materialien im Gebrauch zusammen mit einem niederbrechenden Lenticelmaterial, ist die Herstellung dieser erfindungsgemäßen Phaken Intraokularlinse einfacher und kostengünstiger möglich, als für Phake Linsen mit einem niderbrechenden Lenticelmaterial.

Die Gesamtbrechkraft des Auges wird also dadurch erhöht, daß ein künstliches Medium mit sehr hohem Brechungsindex durch die akkommodative Bewegung der natürlichen Linse angetrieben zunehmend in eine konvexere Form getrieben wird. Dies wird je nach Ausführungsform der Kunstlinse entweder dadurch erreicht, daß Material im Hohlraum der Kunstlinse von der Peripherie in den zentralen Bereich der Linse verlagert wird oder bei konkaven Lenticeln indem die Peripherie abgeflacht und somit die Gesamtbrechkraft des Auges erhöht wird, wodurch eine effektive Nahfokussierung ermöglicht wird.

Zur Kompensation von Brechungsfehlern des Auges können Korrekturen in unterschiedlichen Bereichen der erfindungsgemäßen Phaken Intraokularlinse vorgesehen sein. Die Kompensation von Weitsichtigkeit bzw. Kurzsichtigkeit kann beispielsweise ermöglicht werden durch Modifikationen des ersten und/oder des zweiten Linsenteils sowie durch Modifikationen des Lenticels. Die Modifikation kann in einer veränderten Formgebung des ersten und/oder des zweiten Linsenteils und/oder des Lenticels bestehen oder in einer Änderung des Brechungsindexes eines dieser Bestandteile der Kunstlinse. Die Modifikation kann aber natürlich auch in einer Kombination aus Formveränderung und Änderung des oder der Brechungsindizes bestehen.

Falls nötig können für Randzonen der Kunstlinse, die reflektierende Strukturen aufweisen, reflektionsunterdrückende oder -mindernde Maßnahmen vorgesehen sein, wie z.B. eine Schwärzung oder eine lichtdichte Behandlung des peripheren Bereichs der Linse.

Die erfindungsgemäße Phake intraokulare Kunstlinse wird nachfolgend anhand von in den Figuren dargestellten Ausführungsbeispielen näher beschrieben. Die Erläuterungen erfolgen nur beispielhaft und haben keine beschränkende Wirkung. Die Figuren zeigen rein schematisch:
- Fig.1: eine erste Ausführungsform der erfindungsgemäßen intraokularen Phaken Linse in Fernakkomodation;
- Fig.2: die Phake Linse aus Figur 1 in Nahakkommodation;
- Fig.3: eine zweite Ausführungsform der erfindungsgemäßen intraokularen Phaken Linse in Fernakkomodation; und
- Fig.4: die Phake Linse aus Figur 3 in Nahakkommodation.

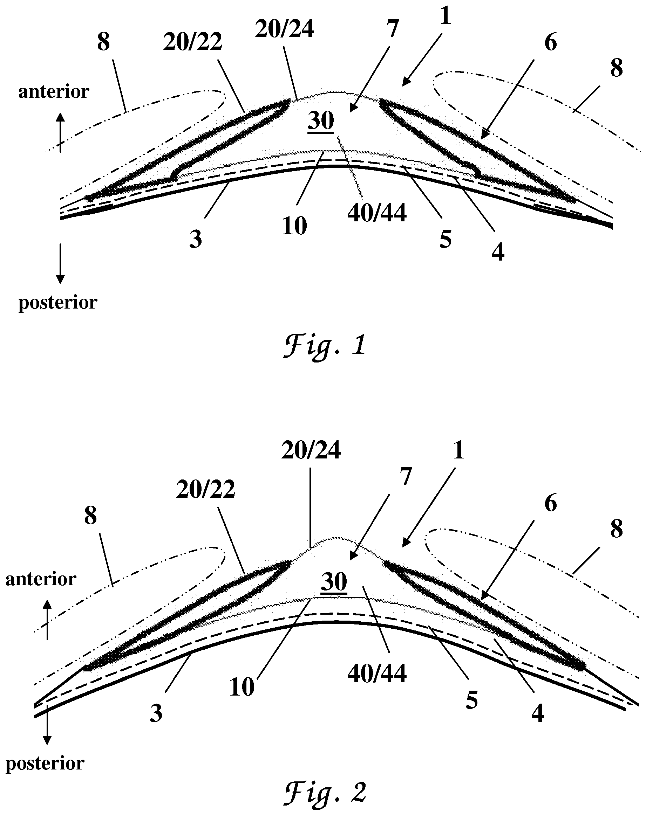

In Fig. 1 und 2 ist eine erste Ausführungsform der erfindungsgemäßen intraokularen Phake Linse 1 gezeigt. Die Kunstlinse 1 ist ausgebildet für die Implantation in ein Auge zusätzlich zur natürlichen Linse 3 und dient der Presbyopiekorrektur. Die Phake Intraokularlinse 1 weist einen ersten Linsenteil 10 auf, der im implantierten Zustand anterior an der natürlichen Linse 3 anliegt, und einen zweiten Linsenteil 20, der dem ersten Linsenteil 10 anterior gegenüberliegt und mit dem ersten Linsenteil 10 zusammen einen Hohlraum 30 einschließt. In dem Hohlraum 30 ist ein Lenticel 40 aus einem verformbaren Medium/Material 44 eingeschlossen, das einen Brechungsindex mindestens von der Höhe des Brechungsindex der natürlichen Linse 3 oder höher aufweist. Die Brechungsindices des ersten Linsenteils 10 und des zweiten Linsenteils 20 liegen etwa im Bereich des Brechungsindexes des Wassers. Sollen zusätzliche Brechungsfehler ausgeglichen werden, können für den ersten und/oder den zweiten Linsenteil 10, 20 auch Materialien mit Brechungsindices über oder unter dem des Kammerwassers gewählt werden.

Damit setzt sich die erfindungsgemäße Kunstlinse 1 aus wenigstens drei funktional unterschiedlichen Komponenten 10, 20, 40 zusammen, die vorzugsweise aus biologisch inerten Materialien gebildet sind, die chemisch nicht miteinander reagieren und die optisch klar sind. Außerdem sind die Materialien vorzugsweise so gewählt, daß der Hohlraum 30 zumindest für das im Hohlraum 30 befindliche Medium 44 diffusionsdicht ist.

Als Medium 44 für den Lenticel 40 wird idealerweise eine Flüssigkeit mit sehr hohem Brechungsindex, vorzugsweise mindestens 1,4 oder darüber, und mit einem spezifischen Gewicht von ca. dem des Kammerwassers gewählt. Der Lenticel 40 ist im Hohlraum 30 derart eingeschlossen, daß eine Materialverlagerung des Lenticelmaterials bzw. des Mediums 44 aus peripheren Anteilen 6 der Linse 1 zum Zentrum 7 hin und zurück entsprechend den akkommodativen Bewegungen der natürlichen Linse 3 möglich ist. Erfindungsgemäß kommen bevorzugt fluide Medien als Lenticelmaterial 44 zum Einsatz, die nicht durch verwendete Linsenmaterialien, wie zum Beispiel das des ersten Linsenteils 10 bzw. des zweiten Linsenteils 20 oder weiterer Linsenbestandteile, hindurch diffundieren können und chemisch nicht mit diesen reagieren. Die Forderung nach biologisch inerten Medien kann in diesem Bereich dahingehend eingeschränkt werden, daß auch solche Substanzen als Medium eingesetzt werden können, die zwar nicht inert aber völlig unbedenklich sind. Gute Erfahrungen wurden beispielsweise mit Silikonöl als Lenticelmaterial 44 gemacht.

Der erste Linsenteil 10 ist aus einer dünnen Schicht eines transparenten Kunstlinsenmaterials, wie beispielsweise aus einem flexiblen Acrylat hergestellt. Er ist vorteilhaft so gestaltet, daß die Dicke und Flexibilität vom Zentrum 7 bis zur Peripherie 6 konstant ist, wobei die Deformierbarkeit bzw. Dehnbarkeit und Flexibilität des ersten Linsenteils 10 vorzugsweise der einer gesunden natürlichen Linse 3 entspricht oder besser ist.

Vorzugsweise liegt die implantierte Phake Linse 1 bzw. ihr erster Linsenteil 10 im implantierten Zustand nicht direkt, sondern durch einen dünnen Flüssigkeitsfilm 4 getrennt an der natürlichen Linse 3 an. Zur Gewährleistung eines stabilen Flüssigkeitsfilms 4 zwischen natürlicher Linse 3 und Kunstlinse 1 kann der erste Linsenteil 10 eine hydrophile Oberflächengestaltung, insbesondere eine hydrophile Beschichtung auf seiner der natürlichen Linse 3 zugewandten Seite aufweisen, wie dies beispielhaft in den Figuren 1 und 2 durch die gestrichelte Linie 5 angedeutet ist.

Der zweite Linsenteil 20 weist in seinen peripheren Bereichen 22 eine relativ geringe Flexibilität beispielsweise Deformierbarkeit/Dehnbarkeit auf. In seinem zentralen Bereich 24 jedoch, der im implantierten Zustand in der optischen Achse liegt, weist der zweite Linsenteil 20 eine hohe Flexibilität bzw. Dehnbarkeit auf. Dieser flexible zentrale Bereich 24 des zweiten Linsenteils 20 bildet einen optisch funktionellen Anteil der Kunstlinse 1. Der periphere inflexible Bereich 22 dient hingegen als Widerlager und bewirkt bei der Verlagerung des Lenticelmaterials von der Peripherie 6 ins Zentrum 7 eine Materialanhäufung im Zentrum. Der flexible Bereich 24 des zweiten Linsenteils 20 wölbt sich aufgrund seiner Dehnbarkeit und des vergrößerten Materialvolumens im Zentrum 7 nach vorne in anteriore Richtung, was zur Brechkrafterhöhung der gesamten intraokularen Kunstlinse 1 führt.

Der inflexible periphere Bereich 22 des zweiten Linsenteils 20 trägt außerdem beträchtlich zur Formstabilität der Kunstlinse 1 bei. Die Formstabilität ist dabei aber nur soweit ausgebildet, daß unter den Bedingungen am Implantationsort keine Verformung der äußeren Anteile der Kunstlinse 1 auftritt, die Linse 1 als Ganzes aber zum Zwecke der Implantation genügend Flexibilität aufweist, daß sie gefaltet werden kann.

Der zweite Linsenteil 20 kann aus dem gleichen Material gefertigt sein wie der erste Linsenteil 10 oder auch aus einem anderen Material. Auch die beiden Bereiche 22, 24 des zweiten Linsenteils 20 können aus dem gleichen Material oder aus unterschiedlichen Materialien gefertigt sein. Sind sie aus dem gleichen Material gefertigt, so können die unterschiedlichen Materialeigenschaften bezüglich Flexibilität, Dehnbarkeit, Deformierbarkeit - abhängig vom gewählten Material - durch unterschiedliche Materialstärken in den unterschiedlichen Bereichen 22, 24 oder durch Bestrahlung z.B. mit elektromagnetischen Strahlen erzeugt werden. Unter diesen Umständen kann der zweite Linsenteil einstückig hergestellt sein. Sind die unterschiedlichen Materialeigenschaften durch unterschiedliche Herstellungsparameter für das gleich Material erzeugt worden oder werden zwei unterschiedliche Materialien für die unterschiedlichen Bereiche 22, 24 eingesetzt, so ist der zweite Linsenteil 20 aus wenigstens zwei Teilen zusammengesetzt.

Der erste Linsenteil 10 und der zweite Linsenteil 20 sind an ihrer Peripherie idealerweise direkt miteinander verbunden, z.B. durch eine Schweißnaht oder beispielsweise weil sie einstückig hergestellt wurden. Die unterschiedlichen Materialeigenschaften können dann wie oben für die verschiedenen Bereiche 22, 24 des zweiten Linsenteils 20 beschrieben (unterschiedliche Materialdicke, Bestrahlung) erzeugt sein. Die Linsenteile 10, 20 und die Verbindung zwischen ihnen ist für das im Hohlraum 30 befindliche Medium 44 des Lenticels 40 undurchlässig.

Statt einer direkten Verbindung zwischen den beiden Linsenteilen 10, 20 kann auch ein Verbindungselement zwischen den beiden Linsenteilen 10, 20 vorgesehen sein, wie beispielsweise eine Ringmembran mit definierter Höhe und Deformierbarkeit (nicht dargestellt). Mit Hilfe einer solchen Ringmembran kann dann erreicht werden, daß die Linsenteile 10, 20 - abhängig von der Verteilung des Mediums 44 im Holraum 30 - zueinender eine variable Distanz innerhalb des Rahmens, den die Höhe der Ringmembran vorgibt, einnehmen können.

Bei Bedarf kann zur Zentrierung der Phake Linse 1 im Auge optional eine Haptik vorgesehen sein (nicht dargestellt). Die Haptik dient allein der Verankerung oder Abstützung der Linse im sogenannten Sulcus. Akkommodative Prozesse in der Kunstlinse 1 werden aber durch die Veränderung des Krümmungsradius der natürlichen Linse 3 gesteuert und nicht über die Haptik.

Die Verstärkung der Akkommodationsfähigkeit der natürlichen Linse wird mit einer erfindungsgemäßen Phaken Intraokularlinse 1 wie sie beispielsweise in den Figuren 1 und 2 gezeigt und oben beschrieben ist, am Implantationsort wie folgt erreicht:
Die Phake Kunstlinse 1 wird so Implantiert, daß der erste flexible Linsenteil 10 an der natürlichen Linse 3 anterior anliegt, und zwar vorzugsweise durch einen dünnen Flüssigkeitsfilm 4 getrennt. Akkommodative Bewegungen der natürlichen Linse 3 werden von dem ersten flexiblen Linsenteil 10 mitvollzogen. Besonders vorteilhaft ist der erste Linsenteil 10 so ausgebildet, daß er die Bewegung der natürlichen Linse 3 zeitlich unmittelbar und in vollem Umfang mitvollziehen kann (wobei mit unmittelbar und in vollem Umfang hier bedeutet, daß mögliche Abweichungen hiervon für den Träger der Phaken Kunstlinse 1 nicht spürbar sind). Der Lenticel 40 steht im Hohlraum 30 in direktem Kontakt mit dem ersten flexiblen Linsenteil 10 und folgt den Bewegungen des ersten Linsenteils 10, was zu einer Materialverlagerung des Lentikelmaterials 44 im Hohlraum 30 der Linse 1 führt. Der Lenticel 40 ist vorzugsweise so ausgebildet und auf den ersten Linsenteil 10 abgestimmt, dass er der Bewegung des ersten Linsenteils 10 in voller Auslenkung folgt. Da der verhältnismäßig starre periphere Bereich 24 des zweiten Linsenteils 20 ein Widerlager bildet, verlagert sich bei einer Nahakkommodation das Material/Medium 44 des Lenticels 40, das sich in der Peripherie 6 zwischen dem flexiblen ersten Linsenteil 10 und dem starren peripheren Bereich 22 des zweiten Linsenteils 20 befindet durch die akkommodative Bewegung in den zentralen optisch funktionellen Teil 7 der Linse 1. Dadurch vergrößert sich das Volumen im Zentrum 7 der Linse 1, was zu einer konvexen Formveränderung des flexiblen zentralen Bereichs 24 des zweiten Linsenteils 20 führt. Daraus resultieren eine Erhöhung der Brechkraft der künstlichen Linse 1 und eine Verstärkung der Akkommodationsfähigkeit der natürlichen Linse 3. Durch eine entsprechende Dimensionierung der einzelnen Linsenkomponenten 10, 20, 22, 24, 30, 32, 40, 44 und die Wahl adäquater Brechungsindizes kann die Brechkrafterhöhung der Kunstlinse 1 an die jeweiligen Bedürfnisse angepasst werden.

Bei zunächst konkaven Lenticeln (nicht dargestellt) kann die Gesamtbrechkraft des Auges dadurch erhöht werden, daß bei der Nahakkommodation die Peripherie 6 der Linse 1 durch die akkommodative Bewegung der natürlichen Linse 3 angetrieben abgeflacht wird, und der hochbrechende Lenticel dadurch zunehmend in eine konvexere Form getrieben wird.

Die in den Figuren 1 und 2 gezeigte Ausführungsform der erfindungsgemäßen Phaken Intraokularlinse 1 wird sehr vorteilhaft als Hinterkammerlinse eingesetzt, was durch eine Andeutung der Iris 8 mit strichpunktierten Linien in den Figuren 1 und 2 angedeutet ist. Hierfür wird die Phake intraokulare Kunstlinse 1 zwischen der natürlichen Linse 3 und der Iris 8 implantiert. Der flexible erste Linsenteil 10 liegt wie bereits beschrieben anterior an der natürlichen Linse 3 an, vorzugsweise durch einen Flüssigkeitsfilm 4 getrennt. Der zweite Linsenteil 20 ist anterior der Iris 8 zugewandt. Er steht mit seinem verhältnismäßig starren peripheren Bereich 24 in direktem Kontakt mit der Iris 8. Bei akkommodativen Schubbewegungen der natürlichen Linse 3 nach vorne dient der starre periphere Bereich 24 des zweiten Linsenteils 20 als Widerlager gegenüber der Iris 8.

In den schematischen Skizzen der Figuren 1 und 2 ist der Effekt der Verstärkung der Restakkommodation dargestellt. In den Zeichnungen sind beide Zustände in Extremstellung dargestellt. Fig. 1 zeigt den Zustand der Fernakkommodation, Fig. 2 den Zustand der Nahakkommodation. Die jeweils stark umrandeten, starren peripheren Bereiche 22 des zweiten Linsenteils 20 ändern ihre Stellung bei der Radienänderung der natürlichen Linse 3 kaum. Der an der natürlichen Linse 3 anliegende flexible erste Linsenteil 10 folgt der akkommodativen Bewegung, wobei die peripheren Anteile des viskosen Lenticels 40 aus hochbrechendem Material 44 ins Zentrum 7 verlagert werden. Hieraus folgt eine Volumenvermehrung im Zentrum 7, die eine Vorwölbung des flexiblen zentralen Bereichs 24 des zweiten Linsenteils 20 der Kunstlinse 1, bzw. eine erhebliche Verringerung des Krümmungsradius auf der anterioren (vorderen) Seite des Lenticels 40 bewirkt. Hieraus resultiert eine wesentliche Erhöhung der Brechkraft der Kunstlinse 1 und funktionell schließlich eine befriedigende Nahakkommodation des Gesamtsystems aus natürlicher Linse 3 und Kunstlinse 1. Wie aus den Figuren ersichtlich, können die Krümmungsradien der anterioren Seiten von hochbrechendem Lenticel 40 und natürlicher Linse 3 erhebliche Differenzen im Verlauf der akkommodativen Bewegung aufweisen. Dabei gilt für den Lenticel 40, je kleiner der Krümmungsradius der Vorderfläche (anteriore Seite) und je höher der Brechungsindex des Lenticelmaterials 44, desto effektiver die Akkommodationsverstärkung.

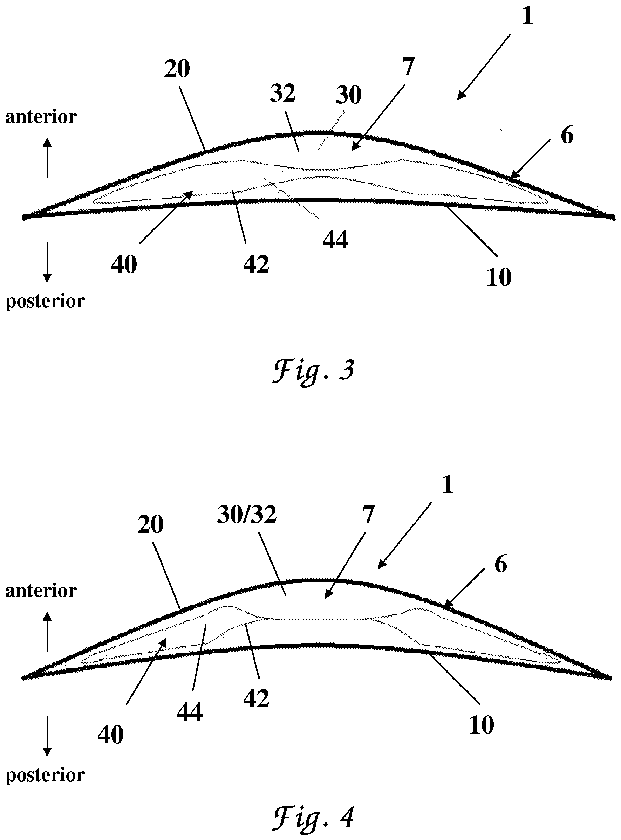

In den Fig. 3 und 4 ist ebenfalls schematisch und zur Verdeutlichung in übertriebener Form eine weitere Ausführungsform einer erfindungsgemäßen Phaken Kunstlinse 1 gezeigt. Fig. 3 zeigt die Kunstlinse 1 im implantierten Zustand bei Fernakkommodation, Fig. 4 zeigt die gleiche Situation bei Nahakkommodation. Aus Gründen der größeren Übersichtlichkeit ist hier allerdings auf die Darstellung der natürlichen Linse verzichtet worden. Die dargestellte Phake Intraokularlinse 1 weist wiederum einen ersten flexiblen Linsenteil 10 auf, der an der natürlichen Linse anliegt und einen dem ersten Linsenteil anterior gegenüberliegenden zweiten Linsenteil 20 sowie einen zwischen den beiden Linsenteilen 10, 20 angeordneten Hohlraum 30. Im Holraum 30 zwischen den beiden Linsenteilen 10, 20 befindet sich ein bikonkav geformter fluider Lenticel 40, der durch eine Membran 42 in seiner äußeren bikonkaven Form definiert ist. Die optisch nicht wirksame Membran 42 ist mit einem transparenten hochbrechenden Medium 44 gefüllt. Neben dem Lenticel 40 mit seiner Membran 42 und seinem hochbrechenden Medium 44 befindet sich im Hohlraum 30 ein weiteres Medium 32, das möglichst wenig brechend ist, das also einen ähnlichen Brechungsindex hat wie die Materialien des ersten Linsenteils 10 bzw. des zweiten Linsenteils 20 bzw. der Membran 42, die vorzugsweise alle einen Brechungsindex im Bereich des Kammerwassers haben. Der Brechungsindex von diesem Medium 32 sowie die Brechungsindices des ersten und des zweiten Linsenteils 10, 20 und/oder der Membran 42 werden bei Konzeption der künstlichen Phake Linse 1 so aufeinander abgestimmt gewählt, dass sie zusammen die Wirkung einer Sammellinse haben und die Differenz zwischen diesen Brechungsindizes und dem Brechungsindex des Lenticels 40 möglichst hoch ist. Durch die akkommodative Bewegung der natürlichen Linse wird das optisch wirksame Material 44 des Lenticels 40 zunehmend aus dem zentralen funktionellen Teil 7 der Kunstlinse 1 in die Peripherie 6 verlagert. Der Lenticel 40 nimmt dadurch positivere Dioptrienwerte an. Bei extremer Nahakkommodation, wie sie in Fig. 4 dargestellt ist, wird das optisch wirksame Material 44 des Lenticels 40, das eine Fokussierung in die Ferne bewirkt, ganz aus dem zentralen Teil 7 der Kunstlinse 1 heraus und vollständig in die Peripherie 6 verlagert. Die vor der Akkommodation für die Ferne eingestellte Kunstlinse 1 verliert dadurch ihre hochbrechende bikonkave Komponente in ihrem zentralen funktionellen Teil 7. Sie verfügt im Zentrum 7 nur noch über konvex wirkende Komponenten, was eine Nahfokussierung bewirkt.

Außer den beiden in den Figuren 1 und 2 einerseits und den Figuren 3 und 4 andererseits gezeigten Ausführungsformen der erfindungsgemäßen Phaken Intraokularlinse 1 sind in der praktischen Umsetzung, noch viele weitere Ausgestaltungen einer akkommodativen Phaken Kunstlinse 1 mit fluidem hochbrechendem Lenticel 40 realisierbar.

Statt des konvexen Lenticels (Figuren 1 und 2) und dem bikonkaven Lenticel (Figuren 3 und 4) können Lenticel mit zunächst parallelen Begrenzungsflächen oder zunächst konkaver Form vorgesehen sein. Das Medium 44 des Lenticels 40 weist vorzugsweise einen Brechungsindex von wenigstens 1,4 auf und hat ein spezifisches Gewicht, das möglichst dem Kammerwasser entspricht, um Ab- oder Auftriebseffekte zu verhindern. Es kann gasförmig, ölig, gelartig, flüssig oder deformierbar fest sein. Als Medium 44 des Lenticels 40 können insbesondere Perflourkarbone oder Silikonöle eingesetzt werden.

Der Lenticel 40 kann unabhängig von seiner Form eine Membran 42 aufweisen, die das Lenticelmaterial/Medium 44 des Lenticels 40 umschließt. Besonders vorteilhaft ist dies aber für parallel und bikonkav ausgebildete Lenticel 40. Die Membran 42 ist deformierbar und umschließt das Medium 44 des Lenticels 40 insbesondere formgebend derart, daß eine Verlagerung des Lenticelmaterials/Mediums 44 innerhalb der Membran 42 möglich und somit eine Formänderung des Lenticels 40 möglich ist. Die Membran 42 kann denselben oder ungefähr denselben Brechungsindex haben wie das Medium 44 des Lenticels 40 oder aber einen Brechungsindex der etwa dem Brechungsindex der umgebenden Materialien entspricht, wie zum Beispiel den gleichen oder ungefähr den gleichen Brechungsindex wie das Material des ersten und/oder des zweiten Linsenteils 10, 20 und/oder des Mediums 32.

Zur Kompensation von Brechungsfehlern des Auges können Manipulationen im/am ersten Linsenteil 10 und oder im/am zweiten Linsenteil 20 und/oder im/am Lenticel 40 vorgesehen sein. Diese können in einer Änderung des Brechungsindexes und/oder in einer Formänderung bestehen.

Die peripheren Bereiche 6 der intraokularen Phaken Linse 1 können reflexmindernd und/oder lichtundurchlässig ausgebildet sein. Sie können beispielsweise geschwärzt sein oder mit einer hydrophilen Beschichtung versehen sein, die bei Wechselwirkung mit wässrigen Flüssigkeiten opak wird.

Der erste Linsenteil 10 kann von seiner Peripherie bis hin zum Zentrum konstante Eigenschaften aufweisen und zwar insbesondere eine konstante Dicke und/oder eine konstante Deformierbarkeit und/oder eine konstante Dehnbarkeit.

Die Deformierbarkeit des ersten Linsenteils 10 kann so auf die natürliche Linse abgestimmt sein, das der erste Linsenteil 10 die akkommodative Bewegungen der natürlichen Linse 3 insbesondere unmittelbar mitvollziehen kann. Sie kann so gewählt sein daß sie derjenigen einer gesunden natürlichen Linse entspricht oder besser ist.

Der zweite Linsenteil 20 kann wie bereits erwähnt, Bereiche 22, 24 unterschiedlicher Deformierbarkeit aufweisen, und zwar insbesondere einen peripheren Bereich 22 mit einer geringen Deformierbarkeit und einen zentralen Bereich 24 mit einer hohen Deformierbarkeit, wobei die Deformierbarkeit von der Peripherie zum Zentrum hin vorzugsweise zumindest in einem Übergangsbereich kontinuierlich zunimmt.

Der zweite Linsenteil 20 kann in seinem peripheren Bereich 22 zur Kraftaufnahme und insbesondere starr ausgebildet sein, so daß er als Widerlager dienen kann.

Die peripheren Bereiche 22 des zweiten Linsenteils 20 können insbesondere dicker ausgebildet sein als der entsprechende zentrale Bereich 24. Wobei es besonders vorteilhaft ist, wenn die Dicke des Materials sich kontinuierlich und stufenlos ändert. Diese Änderung kann von der Peripherie bis ins Zentrum oder zumindest in einem Übergangsbereich zwischen Peripherie und Zentrum kontinuierlich verlaufen. Erhalten werden kann ein solcher Unterschied in der Materialstärke durch entsprechende Formgebung beim Giessen bzw. Formpressen oder nachträglich durch materialabtragende Bearbeitung.

Der zweite Linsenteil 20 kann aber auch durch Bestrahlung, beispielsweise mit elektromagnetischer Strahlung und insbesondere mit Laserstrahlen, in Gänze oder auch nur bereichsweise bezüglich Form und Eigenschaften verändert werden. Veränderbare Eigenschaften sind z.B. Dicke, Verformbarkeit, Flexibilität und Dehnbarkeit. Sehr vorteilhaft kann die Bestrahlung für die Einstellung der Eigenschaften und der Form des peripheren Bereichs 22 des zweiten Linsenteils 20 eingesetzt werden.

Auch der Lenticel kann durch Bearbeitung, insbesondere durch Bestrahlung mit elektromagnetischer Strahlung verändert werden. Insbesondere kann seine Form und damit seine Brechkraft durch Bestrahlung geändert werden. Dies erfolgt vorzugsweise indirekt über den zweiten Linsenteil. So kann beispielsweise durch Bestrahlung der außerhalb des Strahlenganges liegende periphere Bereich 22 des zweiten Linsenteils 20 in seiner Form verändert werden und dadurch auch die Form des Lenticels 40.

Zur Feinjustierung und insbesondere zur Einstellung von Refraktionskorrekturen an einer erfindungsgemäßen Phaken Intraokularlinse kann die Intraokularlinse durch Bestrahlung, und insbesondere Bestrahlung mit elektromagnetischen Strahlen, in ihren Eigenschaften und/oder in ihrer Form verändert werden.

Dem Fachmann ist klar, daß und in welcher Weise sich die beschriebenen Ausführungsformen im Rahmen der Patentansprüche kombinieren lassen. Aus Platzgründen ist es aber nicht möglich alle möglichen und sinnvollen Kombinationen im Detail figürlich darzustellen und/oder zu beschreiben.

Allen Ausgestaltungsvarianten ist gemeinsam, daß der Antrieb der Verformung der Kunstlinse 1 durch eine intakte natürliche Linse 3 erfolgt, sei es durch Änderung des Krümmungsradius der Vorderfläche der natürlichen Linse 3, oder durch eine durch die akkommodative Aktion bedingte Vorverlagerung der natürlichen Linse 3 in Richtung der Iris 8. Weiterhin ist diesen Varianten gemein, daß sie über einen fluiden hochbrechenden Lenticel 40 verfügen, der bei einer Nahakkommodation eine Zunahme an positiver Brechkraft erfährt. Hierbei kann der Lenticel 40 zunächst unterschiedlich geformt in paralleler, konkaver, bikonkaver oder konvexer Ausgestaltung vorliegen und in unterschiedlicher Weise formverändert werden. Die Formveränderung kann durch eine Materialverlagerung des Lenticelmaterials 44 von peripheren Bereichen 6 zum Zentrum 7 hin, durch Materialverlagerung aus dem Zentrum 7 hinaus in periphere Bereiche 6 hinein und/oder durch eine Materialverlagerung innerhalb der Peripherie 6 bzw. aus der Peripherie in optisch nicht genutzte oder nicht nutzbare Teile der Kunstlinse 1 erfolgen.

Für den fluiden Lenticel 40 sollten vorzugsweise hochbrechende Substanzen verwendet werden. Sie sollten in ihrem Brechungsindex möglichst über dem der natürlichen Linse liegen, da mit der Höhe des Brechungsindex die Effektivität der Kunstlinse 1 steigt.

Weiterhin sollte das spezifische Gewicht dieser Substanzen bei Körpertemperatur möglichst dem des Kammerwassers entsprechen, um Probleme durch Auftrieb oder Abtrieb zu vermeiden, da insbesondere an der anterioren Seite der Kunstlinse 1 durch solche Effekte Verzerrungen der Linsengeometrie entstehen können.

Zur Vermeidung osmotischer Effekte, die zu einer Verunreinigung des fluiden Lenticels 40 führen und somit eine Veränderung des Brechungsindex bewirken können, sind hydrophobe bzw. lipophile Substanzen vorteilhaft.

Außerdem sollte die Substanz des Lenticels 40 möglichst nicht toxisch und biologisch inert sein. Sie sollte mit den umgebenden Materialien, beispielsweise dem Material der Linsenteile 10, 20 chemisch nicht reagieren und eine hohe Langzeitstabilität besitzen, da akkommodationsverstärkende Kunstlinsen bevorzugt in der 4 und 5. Lebensdekade implantiert werden und erst im höheren oder hohen Alter beim Auftreten eines grauen Stars explantiert werden.

Diese Anforderungen für eine Lenticelmaterial 44 erfüllen beispielsweise Silikonöle. Da in der operativen Augenheilkunde mit diesen Ölen seit Jahrzehnten Erfahrungen gesammelt wurden, ist es für den Fachmann einfach, ein passendes Silikonöl auszuwählen, daß die spezifischen Anforderungen bezüglich Viskosität und Brechungsindex in Bezug auf die von ihm gewählte Ausführungsform der erfindungsgemäßen Phaken Intraokularlinse erfüllt.

## Patentansprüche

1. Phake Intraokularlinse aus optisch transparentem Material, die dazu ausgebildet ist zusätzlich zu der natürlichen Linse ins Auge implantiert zu werden und insbesondere der Presbyopiekorrektur dient, mit
• einem ersten flächigen Linsenteil (10), der im implantierten Zustand posterior angeordnet ist, und so ausgebildet ist, daß er im implantierten Zustand an der natürlichen Linse (3) des menschlichen Auges anliegen kann, und mit
• einem dem ersten Linsenteil (10) im implantierten Zustand anterior gegenüberliegenden zweiten flächigen Linsenteil (20) sowie mit
• einem Hohlraum (30) zwischen den beiden Linsenteilen (10, 20), wobei der Hohlraum (30) ein optisch transparentes Medium umschließt, wobei
• der erste Linsenteil (10) eine Deformierbarkeit aufweist, die es ihm anliegend an der natürlichen Linse (3) ermöglicht, akkommodative Bewegungen der natürlichen Linse (3) mitzuvollziehen, und
• das im Hohlraum (30) eingeschlossene Medium (44) Bestandteil eines Lenticels (40) ist, und ein im Hohlraum (30) verlagerbares Lenticelmaterial (44) darstellt, **dadurch gekennzeichnet dass**, das im Hohlraum (30) eingeschlossene Medium (44) einen Brechungsindex aufweist, der höher ist als der der umgebenden Medien und
• der zweite Linsenteil (20) zumindest einen deformierbaren Bereich umfasst, der initiiert durch akkommodative Bewegungen der natürlichen Linse (3) in seiner Form bzw. seinem Dehnungszustand änderbar ist.

2. Phake Intraokularlinse nach Anspruch 1,
**dadurch gekennzeichnet, daß**
der erste Linsenteil (10) und der zweite Linsenteil (20) mit seinem verformbaren Bereich konstruktiv so aufeinander abgestimmt sind, daß es bei einer Nahakkommodation der natürlichen Linse (3) zu einer derartigen Materialverlagerung im Lenticel (40) der implantierten Intraokularlinse (1) kommt, daß der Lenticel (40) eine zunehmend konvexere Form annimmt.

3. Phake Intraokularlinse nach Anspruch 1 oder 2,
**dadurch gekennzeichnet, daß**
das Medium (44) des Lenticels (40) einen Brechungsindex von wenigstens 1,4 aufweist und gasförmig, ölig, gelartig, flüssig oder deformierbar fest ist.

4. Phake Intraokularlinse nach einem der Ansprüche 1 bis 3,
**dadurch gekennzeichnet, daß**
der Lenticel (40) eine Membran (42) aufweist, die das Lenticelmaterial/Medium (44) des Lenticels (40) umschließt, wobei der Lenticel (40) insbesondere bikonkav ausgebildet ist und das Lenticelmaterial/Medium (44) vorzugsweise ein Silikonöl ist.

5. Phake Intraokularlinse nach einem der vorangehenden Ansprüche,
**dadurch gekennzeichnet, daß**
der erste Linsenteil (10) von seiner Peripherie (6) bis hin zum Zentrum (7) konstante Eigenschaften aufweist und zwar insbesondere eine konstante Dicke und/oder eine konstante Deformierbarkeit und/oder eine konstante Dehnbarkeit.

6. Phake Intraokularlinse nach einem der vorangehenden Ansprüche,
**dadurch gekennzeichnet, daß**
der erste Linsenteil (10) eine Deformierbarkeit aufweist, die derjenigen einer gut verformbaren natürlichen Linse entspricht oder besser ist.

7. Phake Intraokularlinse nach einem der vorangehenden Ansprüche,
**dadurch gekennzeichnet, daß**
der zweite Linsenteil (20) Bereiche (22, 24) unterschiedlicher Deformierbarkeit aufweist, und zwar insbesondere einen peripheren Bereich (22) mit einer geringen Deformierbarkeit und einen zentralen Bereich (24) mit einer hohen Deformierbarkeit, wobei die Deformierbarkeit von der Peripherie (22) zum Zentrum (24) hin vorzugsweise zumindest in einem Übergangsbereich kontinuierlich zunimmt.

8. Phake Intraokularlinse nach einem der vorangehenden Ansprüche,
**dadurch gekennzeichnet, daß**
der zweite Linsenteil (20) in seinem peripheren Bereich (22) zur Kraftaufnahme ausgebildet ist, so daß er als Widerlager dienen kann.

9. Intraokularlinse nach einem der Ansprüche 7 oder 8,
**dadurch gekennzeichnet, daß**
die peripheren Bereiche (22) des zweiten Linsenteils (20) dicker ausgebildet sind als ein entsprechender zentraler Bereich (24), insbesondere mit einem kontinuierlichen und stufenlosen Übergang.

10. Intraokularlinse nach einem der Ansprüche 7 bis 9,
**dadurch gekennzeichnet, daß**
der zweite Linsenteil (20) ganz oder bereichsweise durch Bestrahlung, insbesondere mit elektromagnetischer Strahlung, bezüglich Form und/oder Eigenschaften verändert ist.

11. Intraokularlinse nach einem der vorangehenden Ansprüche,
**dadurch gekennzeichnet, daß**
der Lenticel (40) durch Bearbeitung, insbesondere durch Bestrahlung mit elektromagnetischer Strahlung in seiner Form und/oder seinen Eigenschaften verändert ist, wobei die Bearbeitung vorzugsweise indirekt über den zweiten Linsenteil (20) erfolgt.

12. Intraokularlinse nach einem der vorangehenden Ansprüche,
**dadurch gekennzeichnet, daß**
zur Kompensation von Brechungsfehlern des Auges Modifikationen im ersten Linsenteil (10) und/oder im zweiten Linsenteil (20) und/oder im Lenticel (40) vorgesehen sind.

13. Intraokularlinse nach einem der vorangehenden Ansprüche,
**dadurch gekennzeichnet, daß**
der erste Linsenteil (10) eine hydrophile Beschichtung (5) zur Stabilisierung eines Flüssigkeitsfilms (4) zwischen Kunstlinse (1) und natürlicher Linse (3) aufweist.

14. Verfahren zur Feinjustierung der Brechkraft einer Phaken Intraokularlinse nach Anspruch 1, **dadurch gekennzeichnet, daß** die Intraokularlinse durch Bestrahlung, insbesondere mit elektromagnetischer Strahlung, in ihren Eigenschaften und/oder in ihrer Form verändert wird.

## Claims

1. A phakic intraocular lens made of optically transparent material, which is designed to be implanted in addition to the natural lens in the eye and is used in particular for presbyopia correction, comprising
- a first planar lens part (10), which is arranged posterior in the implanted state and is designed so that it can rest against the natural lens (3) of the human eye in the implanted state, and comprising
- a second planar lens part (20) opposite anterior to the first lens part (10) in the implanted state, and also comprising
- a cavity (30) between the two lens parts (10, 20), wherein the cavity (30) encloses an optically transparent material,
wherein
- the first lens part (10) has a deformability, which enables it, while resting against the natural lens (3), to co-execute accommodative movements of the natural lens (3), and
- the medium (44) enclosed in the cavity (30) is a component of a lenticel (40) and represents a lenticel material (44) displaceable in the cavity (30),
**characterized in that** the medium (44) enclosed in the cavity (30) has an index of refraction which is higher than that of the surrounding media, and
- the second lens part (20) comprises at least one deformable region, which is variable in its shape and/or its elongation state in a manner initiated by accommodated movements of the natural lens (3).

2. The phakic intraocular lens according to Claim 1,
**characterized in that** the first lens part (10) and the second lens part (20) with its deformable region are structurally adapted to one another so that in the event of a close accommodation of the natural lens (3), a material displacement in the lenticel (40) of the implanted intraocular lens (1) occurs such that the lenticel (40) assumes an increasingly more convex shape.

3. The phakic intraocular lens according to Claim 1 or 2,
**characterized in that** the medium (44) of the lenticel (40) has an index of refraction of at least 1.4, and is gaseous, oily, gel-type, liquid, or deformably solid.

4. The phakic intraocular lens according to any one of Claims 1 to 3,
**characterized in that** the lenticel (40) has a membrane (42), which encloses the lenticel material/medium (44) of the lenticel (40), wherein the lenticel (40) is designed as biconcave in particular and the lenticel material/medium (44) is preferably a silicone oil.

5. The phakic intraocular lens according to any one of the preceding claims,
**characterized in that** the first lens part (10) has consistent properties from its periphery (6) up to the center (7), in particular a consistent thickness and/or a consistent deformability and/or a consistent extensibility.

6. The phakic intraocular lens according to any one of the preceding claims,
**characterized in that** the first lens part (10) has a deformability which corresponds to that of a well deformable natural lens or is better.

7. The phakic intraocular lens according to any one of the preceding claims,
**characterized in that** the second lens part (20) has regions (22, 24) of different deformability, and in particular a peripheral region (22) having a low deformability and a central region (24) having a high deformability, wherein the deformability preferably increases continuously at least in a transition region from the periphery (22) toward the center (24).

8. The phakic intraocular lens according to any one of the preceding claims,
**characterized in that** the second lens part (20) is formed in its peripheral region (22) for force absorption, so that it can be used as an abutment.

9. The intraocular lens according to any one of Claims 7 or 8,
**characterized in that** the peripheral regions (22) of the second lens part (20) are formed thicker than a corresponding central region (24), in particular having a continuous and stepless transition.

10. The intraocular lens according to any one of Claims 7 to 9,
**characterized in that** the second lens part (20) is entirely or regionally modified with respect to shape and/or properties by irradiation, in particular using electromagnetic radiation.

11. The intraocular lens according to any one of the preceding claims,
**characterized in that** the lenticel (40) is modified in its shape and/or its properties by processing, in particular by irradiation using electromagnetic radiation, wherein the processing is preferably performed indirectly via the second lens part (20).

12. The intraocular lens according to any one of the preceding claims,
**characterized in that** modifications are provided in the first lens part (10) and/or in the second lens part (20) and/or in the lenticel (40) to compensate for refraction errors of the eye.

13. The intraocular lens according to any one of the preceding claims,
**characterized in that** the first lens part (10) has a hydrophilic coating (5) for stabilizing a liquid film (4) between artificial lens (1) and natural lens (3).

14. A method for fine adjustment of the refractivity of a phakic intraocular lens according to Claim 1,
**characterized in that** the intraocular lens is modified in its properties and/or in its shape by irradiation, in particular using electromagnetic radiation.

## Revendications

1. Lentille intraoculaire phaque faite d'un matériau optiquement transparent et conformée pour être implantée dans l'oeil en complément du cristallin naturel et à servir en particulier à la correction de la presbytie, avec
• une première partie de lentille (10) formant une surface qui est disposée en position postérieure dans l'état implanté et qui est conformée de façon à pouvoir reposer, dans l'état implanté, sur le cristallin naturel (3) de l'oeil humain, et avec
• une deuxième partie de lentille (20) formant une surface qui fait face à la première partie de lentille (10) du côté antérieur dans l'état implanté et
• une cavité (30) entre les deux parties de lentille (10, 20), laquelle cavité (30) renferme un médium optiquement transparent,
dans laquelle
• la première partie de lentille (10) présente une déformabilité qui lui permet de s'appliquer sur le cristallin naturel (3) et de suivre les mouvements d'accommodation du cristallin naturel (3) et
• le médium (44) renfermé dans la cavité (30) fait partie d'une lenticelle (40) et représente un matériau de lenticelle (44) qui peut se déplacer dans la cavité (30),
**caractérisée en ce que** le médium (44) renfermé dans la cavité (30) présente un indice de réfraction supérieur à celui des média environnants et
• la deuxième partie de lentille (20) comprend au moins une zone déformable dont la forme ou l'état d'étirement peut changer sous l'effet des mouvements d'accommodation du cristallin naturel (3).

2. Lentille intraoculaire phaque selon la revendication 1, **caractérisée en ce que** la première partie de lentille (10) et la deuxième partie de lentille (20) avec sa zone déformable sont d'une construction adaptée l'une à l'autre de sorte que lors d'une accommodation de près du cristallin naturel (3), il se produit un déplacement de matériau dans la lenticelle (40) de la lentille intraoculaire implantée (1) tel que la lenticelle (40) prend une forme de plus en plus convexe.

3. Lentille intraoculaire phaque selon la revendication 1 ou 2, **caractérisée en ce que** le médium (44) de la lenticelle (40) présente un indice de réfraction d'au moins 1,4 et qu'il est gazeux, huileux, gel, liquide ou solide et déformable.

4. Lentille intraoculaire phaque selon l'une des revendications 1 à 3, **caractérisée en ce que** la lenticelle (40) présente une membrane (42) qui entoure le matériau/médium de lenticelle (44) de la lenticelle (40), la lenticelle (40) étant en particulier biconcave et le matériau/ médium de lenticelle (44) étant de préférence une huile de silicone.

5. Lentille intraoculaire phaque selon l'une des revendications précédentes, **caractérisée en ce que** la première partie de lentille (10) présente des propriétés constantes de sa périphérie (6) à son centre (7) et en particulier une épaisseur constante et/ou une déformabilité constante et/ou une extensibilité constante.

6. Lentille intraoculaire phaque selon l'une des revendications précédentes, **caractérisée en ce que** la première partie de lentille (10) présente une déformabilité égale ou supérieure à celle d'un cristallin naturel bien déformable.

7. Lentille intraoculaire phaque selon l'une des revendications précédentes, **caractérisée en ce que** la deuxième partie de lentille (20) présente des zones (22, 24) de déformabilité différente, et en particulier une zone périphérique (22) moins déformable et une zone centrale (24) très déformable, la déformabilité augmentant de la périphérie (22) au centre (24) de façon de préférence continue, au moins dans une zone de transition.

8. Lentille intraoculaire phaque selon l'une des revendications précédentes, **caractérisée en ce que** la deuxième partie de lentille (20) est conformée dans sa zone périphérique (22) de façon à pouvoir absorber des forces, de sorte qu'elle peut servir de contre-appui.

9. Lentille intraoculaire selon l'une des revendications 7 ou 8, **caractérisée en ce que** les zones périphériques (22) de la deuxième partie de lentille (20) sont plus épaisses qu'une zone centrale (24) correspondante, en particulier avec une transition continue et sans paliers.

10. Lentille intraoculaire selon l'une des revendications 7 à 9, **caractérisée en ce que** la deuxième partie de lentille (20) a une forme et/ou des propriétés modifiées par une irradiation entièrement ou par zones, en particulier à l'aide d'un rayonnement électromagnétique.

11. Lentille intraoculaire selon l'une des revendications précédentes, **caractérisée en ce que** la lenticelle (40) a une forme et/ou des propriétés modifiées par un traitement, en particulier par une irradiation avec un rayonnement électromagnétique, le traitement étant effectué de préférence de façon indirecte, en passant par la deuxième partie de lentille (20).

12. Lentille intraoculaire selon l'une des revendications précédentes, **caractérisée en ce que** pour compenser les erreurs de réfraction de l'oeil, des modifications sont prévues dans la première partie de lentille (10) et/ou dans la deuxième partie de lentille (20) et/ou dans la lenticelle (40).

13. Lentille intraoculaire selon l'une des revendications précédentes, **caractérisée en ce que** la première partie de lentille (10) présente un revêtement hydrophile (5) pour stabiliser un film de liquide (4) entre le cristallin artificiel (1) et le cristallin naturel (3).

14. Procédé pour l'ajustement fin du pouvoir de réfraction d'une lentille intraoculaire selon la revendication 1, **caractérisé en ce que** les propriétés et/ou la forme de la lentille intraoculaire sont modifiées par exposition à un rayonnement, en particulier à un rayonnement électromagnétique.
